Europäisches Patentamt

European Patent Office

Office européen des brevets

(19)

(11) Publication number: **0 179 583**

**A1**

(12) **EUROPEAN PATENT APPLICATION**

(21) Application number: **85307025.8**

(22) Date of filing: **01.10.85**

(51) Int. Cl.⁴: **A 61 K 47/00**

(30) Priority: **04.10.84 US 657710**

(43) Date of publication of application:
**30.04.86 Bulletin 86/18**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065(US)**

(72) Inventor: **Pope, David G.**
**19 Tall Timber Road**
**Middletown New Jersey 07748(US)**

(72) Inventor: **Wilkinson, Paul K.**
**36 Totem Road**
**Freehold New Jersey 07728(US)**

(74) Representative: **Crampton, Keith John Allen et al,**
**D YOUNG & CO 10 Staple Inn**
**London WC1V 7RD(GB)**

(54) **A system for enhancing the water dissolution rate and solubility of poorly soluble drugs.**

(57) Novel anhydrous compositions comprise a poorly water-soluble drug and a surfactant having enhanced dissolution rate and solubility. The drug-surfactant ratio in the composition is such that dissolution and solubility of the drug are enhanced. The compositions disclosed herein are useful in the treatment of patients (human and veterinary) as well as domestically and in agriculture.

EP 0 179 583 A1

1

# A SYSTEM FOR ENHANCING THE WATER DISSOLUTION RATE
## AND SOLUBILITY OF POORLY SOLUBLE DRUGS

The invention relates to the solubilization of drugs in water. More particularly, the invention concerns the preparation of compositions comprising a poorly water-soluble drug and a surfactant by a process that enhances the water dissolution rate and solubility of the drugs by in situ micelle formation.

Many drugs, particularly poorly water-soluble or water-insoluble compounds such as ivermectin, abamectin and griseofulvin, exhibit poor, incomplete, and/or irregular absorption when administered to humans or animals because of their irregular dissolution rate. To increase dissolution rate, prior formulation systems utilized reduction in particle size by milling, by coprecipitation with a water-soluble carrier, by formation of a solid solution in a water-soluble carrier, by formation of a suitable more soluble salt, by formulation with a buffer salt, by the addition of small amounts of surfactant to improve wettability, and by adsorption onto a high-surface-area silica.

The literature contains numerous references involving processes and compositions for enhancing dissolution rate and solubility of poorly water-soluble drugs in aqueous media. Specifically, U.S. Patent Specification US-A-4,344,934 discloses compositions comprising a poorly soluble drug, a water-soluble polymer and a wetting agent that increases bio-availability of the drug. In this document polymers are used in conjunction with a wetting agent.

The use of surfactants to improve dissolution rate has been limited to reduction in the interfacial tension between the drug and dissolution media by the addition of surface-active agent to the dissolution media. The addition of surfactants improves the effective surface area of the drug by enabling the solution to wet the drug more effectively. For example, Finholt and Solvang, J. Pharm. Sci. 57: 1322 (1968) showed that the dissolution rate of phenacetin (a hydrophobic drug), was increased markedly by the addition 0.01% of polysorbate 80, a surfactant. The effect of polysorbate 80 on the rate of dissolution of the phenacetin was due only to a small extent to its solubilizing power. It was caused mainly by its ability to decrease the interfacial tension between the substance and its dissolution medium.

Also Finholt and Solvang, Meddelelser fra Norsk, Farmacutisk Selskap, 31, 101(1969), have admixed low concentrations of surfactant with a solid drug powder or formulation, achieving the improved dissolution by a wetting phenomenon. For example, improved dissolution of phenacetin tablets resulted from dissolving polysorbate 80 in the granulating solution or spraying it as an alcoholic solution onto the dried granules.

The ability of surfactants to micellize poorly water-soluble drugs is widely known. When a surfactant is dissolved in water at very low concentrations, a fraction of it will be adsorbed at the air-water interface, and the remainder will reside in the bulk in the form of monomers. As the concentration is increased, a level is reached where the interface becomes saturated with surface-active agent and, usually simultaneously, the limiting solubility of monomer in the bulk is approached. At this concentration an unusual phenomenon occurs. Rather than precipitate, the monomers in the bulk tend to form colloidal aggregates termed micelles, which consist of 50 to 150 molecules or ions of surface-active agent. The concentration at which aggregation occurs is called the critical micelle concentration or CMC. The molecules in the micellar unit have a very definite orientation; the hydrocarbon or nonpolar portion is oriented to the centre of the micelle and is shielded from the aqueous solution. In essence a micellar solution consists of many nonpolar "droplets", which can function as a discrete phase and thereby interact with or "dissolve" drugs that would normally be insoluble in aqueous systems. This phenomenon is termed micellar solubilization. Thus, all existing literature on micellar solubilization utilizes preformed micelles in aqueous solution, i.e., the surfactant is first dissolved and then the poorly soluble drug is dissolved in the preformed micelle. The product is then stored in an aqueous medium, dispensed, and used as such.

In accordance with this invention, there is provided an anhydrous composition comprising a poorly water-soluble drug and a surfactant. The drug (e.g. ivermectin, abamectin, thiabendazole and clorsulon) is combined by suitable techniques with appropriate surfactant systems to form anhydrous products which, upon addition to water, result in solution of the drug by in situ micelle formulation. The solubility and dissolution rate of the drug can be enhanced in accordance with the present invention.

Because of the absence of water, a concentrate suitable for dilution, without the bulk storage and handling problems associated with the equivalent more bulky aqueous system, can be obtained.

Compositions of the invention have a favourable storage mode for drugs that are susceptible to degradation in aqueous solution and simultaneously maintain the ability to obtain a clear aqueous solution upon addition to aqueous media before use.

Compositions of the invention can be administered to the human and or animal body or used in the environment, either as such or following ready dissolution into an aqueous delivery medium.

The novel compositions of the invention involve combining the poorly water-soluble drug with the surfactant in appropriate ratios and by an appropriate method that results in the formation of an anhydrous product. This product can be a solid or liquid and, on addition to water, results in a rate of solution equivalent to the surfactant and an improved water-solubility of the drug such that the composition is totally usable with water in all proportions. This process provides a formulation system that allows pharmaceutical formulation of anhydrous solid or liquid product, yet allows dissolution and solubility in water via in situ micelle formation, whereas previous micelle systems were prepared by first dissolution of the surfactant and then dissolution of the poorly water-soluble drug into the already formed micelle system.

To further decribe the novel nature of this invention and its ability to improve dissolution rate and solubility of poorly water-soluble drugs, the rate of dissolution and solubility of a poorly water-soluble drug from prior systems (Systems 1-5) are compared with the system of this invention (System 6) in Table 1.

4

## TABLE 1

| FORMULATION | CONSEQUENCE |
|---|---|
| 1) Drug & water added | Very poor solubility and dissolution rate. |
| 2) Drug & (water & low concentration surfactant) | Water solubility still poor, improved dissolution rate. |
| 3) Drug & (water & surfactant at a concentration above the critical micelle concentration) | Improved water solubility and improved dissolution rate. |
| 4) (Drug & low concentration of surfactant admixed) & water | Water solubility still poor, improved dissolution rate. |
| 5) (Drug & physical mix of surfactant in sufficient amount to be above CMC on dilution with water) & water | Water solubility improved, improved dissolution rate. Dissolution rate will generally be less than that described in (3). |
| 6) (Drug & surfactant reacted as described in this application) & water | Water solubility improved. Dissolution rate faster than all other systems. Dissolution rate dependent not on the insoluble or poorly soluble drug but on the rate of solution of the water soluble surfactant selected. |

The compositions of this invention (System 6 above) comprise a poorly water-soluble drug and surfactant and optionally conventional excipients and preservatives, the drug-surfactant ratio being such that a clear infinitely diluable micelle solution is formed with the addition of an aqueous medium. The compositions are prepared by one of three processes, which are generally described below:

### a. Micelle/Evaporation Process

This process involves forming a traditional micelle solution with the drug and surfactant, e.g. sodium lauryl sulphate, sodium desoxycholate or Lexaine (cocoamido alkyl betaine), either by simple dissolution of both in water or by the addition of an aqueous alcoholic solution of the drug to the surfactant solution. The surfactant of choice should have a high micellization capacity and be a solid at normal room temperature. The micelle solution is then dried, e.g. by drum drying, evaporation on a Rotovac, or lyophilization. The solid obtained is an anhydrous micelle system with the drug locked in and distributed throughout the surfactant system in such a way that upon addition of water, the aqueous micelle solution is again readily obtained.

### b. Solution/Adsorption Process

The poorly water-soluble drug is dissolved in a liquid surfactant such as a polysorbate, a polyoxyethylene polyol, or a sorbitan. This solution is adsorbed onto an ad-absorber such as one of those solid under the trade mark Cab-O-Sil or Accurel Powder, to form a dry flowable powder. The resulting solid anhydrous product, when placed in an aqueous medium, dissolves, forming in situ an aqueous micelle solution. An organic cosolvent or water-miscible cosolvent may be added to the surfactant to increase drug solubility in the anhydrous system.

### c. Solution/Melt Process

The poorly water-soluble drug is dissolved in a molten surfactant having a melting point below that of the degradation temperature of the drug but above normal storage temperatures, e.g. a polyoxyethylene polyol, polyoxyalkylated isostearyl alcohol or ethoxylated tallowate. The solid anhydrous system, when added to an aqueous medium, will dissolve forming in situ an aqueous micelle solution.

Various active agents provide beneficial effects when administered to patients in compositions in accordance with the present invention. Such agents are exemplified by, but not limited to, the following:

(a) ß-blockers, such as propanolol, bupranolol, metoprolol, nadoxolol, sotalol, alprenolol, oxprenolol, carteolol, labetalol, atenolol, pindolol, timolol and timolol maleate.

The preferred ß-blockers are timolol, bupranolol, timolol maleate and propanolol.

(b) antimicrobial agents, such as antibacterial, antifungal and antiviral agents, e.g. lincomycin; clindamycin; tetracycline, oxytetracycline, chlorotetracycline and other tetracycline-type antibiotics; erythromycin; 2-thiopyridine N-oxide; halogen compounds, especially iodine and iodine compounds; cephalosporins, including any of the many new forms of these ß-lactam antibiotics, e.g. penicillin, penicillin G, methacillin, carbenicillin, ticaricillin, cephalosporin C, cefazolin, cephapirin, cephaloridine, cephalothin, cephanone, cefamandole, cefaparole, cefoxitin, cephacetrile, cefmetazole, cefoxitin, cefuroxime, cefotaxime, T-1551, and the oxacephalosporin S-6059; sulphonamide antibacterials; and streptomycin.

The preferred agents are linacomycin, tetracycline, erythromycin, penicillin, penicillin G, cefoxitin, streptomycin, carbenicillin, cephapirin, cephalosporin C and cephanone.

(c) steroidal anti-inflammatory agents, such as the corticosteroids hydrocortisone, hydrocortisone 17-valerate, hydrocortisone 17-butyrate, hydrocortisone 21-acetate, betamethasone valerate, triamcinolone acetonide, fluocinonide, desonide, fluocinolone acetonide, dexamethasone, dexamethasone 21-phosphate, prednisolone, prednisolone 21-phosphate, haloprednone, cortisone acetate, hydrocortisone cyclopentylpropionate, cortodoxone, flucetonide, fludrocortisone acetate, flurandrenolone acetonide, medrysone, amcinafal, amcinafide, betamethasone, betamethasone benzoate, chloroprodnisone acetate, clocortolone acetate, descinolone acetonide, desoximetasone, dichlorisone acetate, difluprednate, flucloronide, flumethasone, flumethasone pivalate, flunisolide acetate, fluocortolone, fluorometholone, fluperolone acetate, fluprednisolone, fluprednisolone valerate, meprednisone, methyl prednisolone, paramethasone acetate, prednisolamate, prednisone, prednival, triamcinolone, triamcinolone hexacetonide, cortivazol, formocortal and nivazol.

The preferred agents are hydrocortisone, hydrocortisone 21-acetate, betamethasone, betamethasone valerate, prednisolone, prednisolone 21-phosphate, cortisone acetate, fludrocortisone acetate, triamcinolone and nivazol.

(d) Non-Steroidal anti-inflammatory agents, such as indomethacin, naproxen, fenoprofen, ibuprofen, alcolfenac, phenylbutazone, mefenamic acid, sulindac, desoxysulindac, diflunisal, aspirin, salicylamide, salicylic acid, flufenisal, salsalate, triethanolamine salicylate, aminopyrine, antipyrine, oxyphen-butazone, apazone, cintazone, flufenamic acid, clonixeril, clonixin, meclofenamic acid, flunixin, colchicine, demecolcine, allopurinol, oxypurinol, benzylamine hydrochloride, dimefadane, indoxole, intrazole, membrane hydrochloride, paranylene hydrochloride, tetrydamine, benzindopyrine hydrochloride, fluprofen, ibufenac, ketoprofen, naproxol, fenbufen, cinchophen, diflumidone sodium, fenamole, flutiazin, metazamide, letimide hydrochloride, nexeridine hydrochloride, octazamide, molinazole neocinchophen, nimazole, proxazole citrate, tesicam, tesimide, tolmetin, tramadol and triflumidate.

The preferred agents are indomethacin, naproxen, fenaprofen, sulindac, ibuprofen, diflunisal, aminopyrine, antipyrine, nimazole, tramadol, fluprofen and demecolcine.

(e) Antihypertensive agents such as clonidine and α-methyldopa, and antiangina such as propanolol hydrochloride erythrityl tetranitrate, pentaerythritol tetranitrate, isosorbide dinitrate and dioxyline phosphate; vasodilator agents such as nitroglycerin, erythritol tetranitrates, isosorbide dinitrate, mannitol hexanitrate, pentaerythrityl tetranitrate, papaverine and dipyridamole.

The preferred agents are methyldopa, clonidine and antianginas such as propanolol hydrochloride, erythrityl tetranitrate or dioxyline phosphate.

(f) Sex hormones such as estrogens, androgens and progestins, especially the natural sex hormones estradiol, testosterone and progesterone.

The preferred agents are estrogen, progestin, androgen, testosterone and progesterone.

(g) Muscle relaxants such as succinylcholine chloride, baclofen, dantrolene sodium, metaxalone, cyclobenzaprine hydrochloride and diazepan.

(h) Antiasthma agents, such as theophylline, terbutaline sulfate, dyphyline, guaifenesin and cromoglycic acid and its prodrugs [described, for example, in International Journal of Pharmaceutics, 7, 63-75 (1980)]. Because of its short half-life, cromoglycic acid is an especially desirable candidate for formulation with polyvinyl alcohol in accordance with the present invention.

(i) Antimetic agents, such as pipamazine, chlorpromazine and dimenhydrinate.

(j) Antidepressant agents, such as protriptyline hydrochloride, amitriptyline, perphenazine and amitriptyline hydrochloride, chlordiazepoxide hydrochloride, phenizine sulfate and doxepin hydrochloride.

The preferred agents are protriptyline hydrochloride, chlordiazepoxide hydrochloride, amitriptyline and doxepin hydrochloride.

(k) Diuretics such as aldactone, diuril dyazide, enduron, hydrochlorothiazide and bretic.

The preferred agents are aldactone, diuril and hydrochlorothiazide.

(l) Vasodilator agents such as nitroglycerin, erythritol tetranitrate, isosorbide dinitrate, mannitol hexanitrate, pentaerythrityl, papaverine and dipyridamole.

The preferred agents are nitroglycerin, erythritol tetranitrate, mannitol hexanitrate and papaverine.

(m) Antiparasitic agents such as ivermectin, avermectin, abamectin, thiabendazole, milbemycin, albendazole and the like.

The preferred agents are ivermectin, abarmectin, thiabendazole, and milbemycin.

Those skilled in the art will realize that the type of beneficial agent used is not critical and that any beneficial agent can be used in accordance with the practice of this invention as long as it is soluble in the surfactant.

The surfactants useful in the practice of the invention are not critical. However, the preferred surfactants are those which result in a low surfactant to drug ratio. Representative surfactants which can be employed in the practice of the invention are polysorbates such as grades 20 and 80, polyoxyethylene polyols such as Butronic L-1, and Pluronic 25R4, sodium lauryl sulphate, sodium desoxycholate, sorbitans such as Span 20 & 80, polyoxyalkylated isostearyl alcohols such as Arosurf$^R$ grades 66-E2, E10 and E20 and the like polyoxyethylene alkyl ethers such as Brij$^R$ 35, 56, and 96 and the like, polyoxyethylene polyol fatty acid esters such as Arlatone$^R$ T, polyoxyethylene fatty glycerides such as Arlatone G, cocoamido alkyl betaines such as Lexaine$^R$, dioctyl sodium sulfosuccinate, ethoxylated tallowates such as Varonic$^R$ LI grades 42 and 48, C-cetyl and C-decyl betaines and stearyl dimethyl benzyl ammonium chloride, ether amine salts such as Surfac$^R$ grades P24M, P14B and 18-EHP and the like.

The pharmaceutical compositions containing the active ingredient may be in a form suitable for oral use, for example, as tablets, troches, lozenges, dispersible powders or granules, or hard or soft capsules. Compositions intended for oral use may be prepared according to any method known to the art for the manufacture of pharmaceutical compositions and such compositions may contain one or more sweetening agents, flavouring agents, colouring agents and/or preserving agents in order to provide pharmaceutically elegant and palatable preparation.

Formulations for oral use include tablets which contain the active ingredient in admixture with non-toxic pharmaceutically acceptable excipients. These excipients may be, for example, inert diluents, such as calcium carbonate, sodium chloride, lactose, calcium phosphate or sodium phosphate; granulating and disintegrating agents, for example, maize starch, or alginic acid; binding agents, for example, starch, gelatin or acacia, and lubricating agents, for example, magnesium stearate, stearic acid or talc. The tablets may be uncoated or they may be coated by known techniques to delay disintegration and absorption in the gastrointestinal tract and thereby provide a sustained action over a longer period.

Formulations for oral use may also be presented as hard gelatin capsules wherein the active ingredient is mixed with an inert solid diluent, for example, calcium carbonate, calcium phosphate or kaolin, or as soft gelatin capsules.

The pharmaceutical composition of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug, for example, cocoa butter and polyethylene glycols.

For topical use, creams, ointments, jellies, or the like containing the drug are employed according to methods recognized in the art.

Naturally, the therapeutic dosage range for the drugs employed in the practice of the invention will vary with the size and needs of the patients and the particular pain or disease symptoms being treated. However, generally speaking, the following dosage guidelines will suffice. Orally, the therapeutic dose required for a drug will range from 0.001 to 50 gram per dose preferably from 0.01 to 25 gram per dose.

The amount of active ingredient that may be combined with the carrier materials to produce a single dosage form will vary depending upon the host treated and the particular mode of administration or use. For example, a formulation intended for oral administration of humans may contain from 5 mg to 5 g of active drug per dose compounded with an appropriate and convenient amount of carrier material which may vary from 5 to 95 percent of the total composition.

It will be understood, however, that the specific dose level for any particular patient or any other use will depend upon a variety of factors including the activity of the specific drug, the age, body weight, general health and sex of the patient, diet, time of administration, route of administration, rate of excretion, drug combination and the severity of the particular disease undergoing therapy.

Generally, the ratio of drug to surfactant combination ranges from 1:0.05 to 1:20 (preferably from 1:1 to 1:10) by weight and the combination constitutes from 10% to 100% (preferably from 40%-98%) of the total formulation.

The following examples illustrate but do not limit various compositions of the invention and their preparation. In the Examples, mesh sizes are U.S. standards. Parts and percentages unless otherwise stated are by weight.

## Micelle/Evaporation Process

### EXAMPLE 1

A solution comprising 33 parts ivermectin (10 percent active) and 66 parts sodium lauryl sulphate (SLS) (100 percent active) in a 30% (v/v) aqueous methanolic solution is prepared by stirring the above in a suitable container until everything is dissolved. The solution is transferred to the receiving vessel of a Rotovap apparatus. The vessel is placed in a 40°C water bath and sufficient vacuum applied to effect distillation of the methanol and water. The dry powder is collected and passed through a 40 mesh screen. This dry powder may be used as such or as processed in Example 10.

### EXAMPLE 2

The solution of Example 1 (ivermectin, SLS and aqueous methanol) is subdivided and placed in shallow metal or glass vessels. The vessels containing the solution are placed into a commercial lyophilizer. The trays are chilled to -30 °C or below and the vacuum of the lyophilizer is increased and the temperature permitted to rise according to the lyophilizer manufacturer's instruction until a dry powder is produced. The dry powder cake is collected and passed through a 40-mesh screen. This dry powder may be used as such or as processed as in Example 10.

### EXAMPLE 3

Following the procedure of Example 2, the same product was prepared by the addition of 0.1 part disodium edetate and 0.1 part butylated hydroxytoluene to the ivermectin, SLS and aqueous methanol solution. This dry powder may be used as such or as processed in Example 10. Also, when other active agents and surfactants are substituted for the ivermectin and sodium lauryl sulphate of Examples 1 or 2, the corresponding anhydrous product is obtained.

### EXAMPLE 4

Examples 1-3 are repeated with the exception that sodium desoxycholate or Lexaine P-100 is used in an appropriate drug:surfactant ratio instead of sodium lauryl sulphate. This dry powder may be used as such or as processed as in Example 10.

### Solution/Ad-Absorption Process

### EXAMPLE 5

A solution comprising by weight 7.5 parts ivermectin (100 percent active) in 100 parts of a non-aqueous solution comprising by volume 28.5 parts glycerol formal and 71.5 parts polysorbate 80 is prepared by stirring in a suitable container until dissolved. The solution may be used as such or as in Example 11.

### EXAMPLE 6

A solution comprising by weight 7.5 parts abamectin (100 percent active) in 100 parts of a non-aqueous solution comprising by volume 36 parts glycerol formal and 64 parts polysorbate 80 is prepared by stirring in a suitable container until dissolved. The solution may be used as such or as in Example 11.

When other active agents and surfactants are substituted for ivermectin and polysorbate 80, respectively, as in Examples 5 or 6, there is obtained the corresponding product.

## Solution/Melt process

### EXAMPLE 7

A solution comprising by weight 7 parts ivermectin (100 percent active) in L-1 Butronic is prepared by gently heating above the Butronic melting point (approximately 45°-50°C) in a suitable container. Ivermectin is added and stirred until dissolved. The solution is allowed to cool to a waxy solid at room temperature.

### EXAMPLE 8

A solution comprising by weight 7 parts ivermectin (100 percent active) in Li-42 Varonic is prepared by gently heating above the Varonic melting point (approximately 35°-45°C) in a suitable container. Ivermectin is added and stirred until dissolved. The solution is allowed to cool to a soft solid at room temperature.

### EXAMPLE 9

A solution comprising by weight 7 parts ivermectin (100 percent active) in 66E20 Arosurf is prepared by gently heating above the Arosurf melting point (approximately 35°-45°C) in a suitable container. Ivermectin is added and stirred until dissolved. The solution is allowed to cool to a soft solid at room temperature.

When other active agents and surfactants are substituted for ivermectin and Butronic or Varonic, respectively, as in Examples 7-9, there is obtained the corresponding product.

Formulation Dissolution Rate
## EXAMPLE 10

The powder produced in Examples 1-4 are directly compressed into tablets by conventional tableting methods.   The dissolution of said tablets to yield a clear infinitely diluable aqueous solution of ivermectin is very rapid (15 minutes with gentle agitation).   Compressed tablets of ivermectin alone do not dissolve or disintegrate within 3 days.   The addition of a dry admixture of ivermectin and SLS to deionized distilled water resulted in dissolution rates less than those observed with the invention.

## EXAMPLE 11

37 parts of each of the solutions produced in Examples 5 and 6 is dry blended with 1 part of Accurel powder (50-75% void space).   This mixture is then blended with 12 parts of Cab-O-Sil to produce a dry free-flowing powder which may be used as such or tableted as in Example 10.   The addition of ivermectin, alone or dissolved in glycerol formal, to an aqueous surfactant solution results in dissolution rates less than those observed with the invention.

## EXAMPLE 12

The thermoplastic solids produced in Examples 7-9 are added to deionized distilled water.   This mixture is stirred to affect a clear infinitely diluable solution of ivermectin.   At temperatures below the melting point of the solid produced in Examples 7-9 the rate is diminished compared to that observed when that solid is present in the molten state. This dissolution is complete at all proportions of water as shown in Example 10. The dissolution rate of ivermectin into an aqueous solution of an equivalent amount of thermoplastic surfactant is significantly slower as compared to the method of the invention.

## Stability

### EXAMPLE 13

A solution of 5% w/v ivermectin is prepared by dissolving the powder produced in Example 3 in sufficient deionized distilled water. This solution is stored at elevated temperature (40°C and 50°C) in a controlled environment chamber for 8 weeks. The dry powder produced in Example 3 is also stored at 25°C and 50°C. Table II presents a comparison of the stability of ivermectin in the dry state and as a micelle solution.

### TABLE II

| Sample | Time/ Temperature | % loss | Aqueous Composition |
|---|---|---|---|
| Solution | 8 wks/40°C | 16.5% | yes |
| | 2 wks/50°C | 62% | yes |
| Solid | 12 wks/50°C | 0% | no |
| | 12 mos/25°C | 0% | no |

It has been demonstrated that the anhydrous _in situ_ forming micelle powder improves the stability and storage capacity of ivermectin.

## Solubility

### EXAMPLE 14

A solution of 5% (50 mg/ml) w/v ivermectin is prepared by dissolving the product in Examples 1-5 and 7-9 in sufficient deionized distilled water. A mixture of an equivalent amount of ivermectin in deonized distilled water is only marginally soluble (1-4 mcg/ml). Therefore, the solubility of a poorly water soluble drug is enhanced (50,000 mcg/ml).

18

CLAIMS

1. An anhydrous composition comprising a poorly water-soluble drug and a surfactant.

2. A composition as claimed in Claim 1 in which the drug is a β-blocker, antimicrobial agent, anti-inflammatory agent, non-steroidal anti-inflammatory agent, antihypertensive agent, sex hormone, muscle relaxant, antiasthma agent, antiemetic agent, antidepressant, diuretic, vasodilator or antiparasitic agent and the surfactant is a polysorbate, a polyoxyethylene polyol, sodium lauryl sulphate, sodium desoxycholate, a sorbitan, a polyoxyalkylated isostearyl alcohol, a polyoxyethylene alkyl ether, a polyoxyethylene polyol fatty acid ester, a polyoxyethylene fatty glyceride, a cocoamido alkyl betaine, a dioctyl sodium sulphosuccinate, an ethoxylated tallowate, a C-cetyl or C-decyl betaine, stearyl dimethyl benzyl ammonium chloride or an ether amine salt.

3. A composition as claimed in Claim 2 in which the drug is timolol, propanolol, bupranolol, cefoxitin, erythromycin, lincomycin, hydrocortisone, betamethasone, prednisolone, indomethacin, sulindac, diflunisal, methyldopa, clonidine, estrogen, androgen, progestin, cyclobenzaprine, baclofen, diazepan, theophyline, dyphyline, pipamazine, chlorpromazine, protriptyline, amitriptyline, aldactone, diuril, hydrochlorothiazide, ivermectin, thiabendazole, avermectin, abamectin, nitroglycerin or papaverine, and the surfactant is polysorbate 80, a polyoxyethylene polyol, sodium lauryl sulphate or sodium desoxycholate.

4. A composition as claimed in any preceding claim in which the ratio of the weight of drug to that of surfactant ranges from 1:0.05 to 1:20 and the drug-surfactant combination constitutes from 10 to 100% by weight of the total formulation.

5. A composition as claimed in Claim 4 in which the said ratio ranges from 1:1 to 1:10 and the said combination constitutes from 40 to 98% of the total formulation.

19

0179583

6.      A composition as claimed in any preceding claim, for use in the treatment of a medical disorder.

7.      The use of a composition as claimed in any one of Claims 1 to 5 in domestic or agricultural applications where the drug component is known for such use.

8.      A process for preparing a composition as claimed in any one of Claims 1 to 5 comprising appropriately dissolving the drug into the surfactant at a temperature in the range from room temperature for liquid surfactants to temperatures no greater than 10 °C above the melting point of solid surfactants.

9.      The process as claimed in Claim 8 including the further step of dissolving the solution obtained in an aqueous or aqueous alkanolic solvent and evaporating to a dry product at a temperature in the range -30 °C to +60 °C.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | CHEMICAL ABSTRACTS, vol. 101, no. 2, 9th July 1984, page 293, no. 12070q, Columbus, Ohio, US; N.M. SANGHAVI et al.: "Effect of various surface active agents on dissolution rate of drug phenytoin", & INDIAN DRUGS 1984, 21(5), 206-8 <br> * Abstract * | 1-9 | A 61 K 47/00 |
| X | CHEMICAL ABSTRACTS, vol. 98, no. 6, 7th February 1983, page 356, no. 40512y, Columbus, Ohio, US; N.M. SANGHAVI et al.: "Effect of surface active agents on drug dissolution of tolbutamide and phenobarbitone", & INDIAN DRUGS 1982, 19(11), 421-4 <br> * Abstract * | 1-9 | |
| X | CHEMICAL ABSTRACTS, vol. 78, no. 8, 26th February 1973, page 272, no. 47718r, Columbus, Ohio, US; J.A. REES et al.: "Effects of micellar concentrations of polysorbate 20 on the dissolution rate of salicylic acid", & J. PHARM. PHARMACOL. 1972, 24(SUPPL.), 154P <br> * Abstract * <br><br> ---     -/- | 1-9 | TECHNICAL FIELDS SEARCHED (Int Cl 4) <br><br> A 61 K |

The present search report has been drawn up for all claims

| Place of search | Date of completion of the search | Examiner |
|---|---|---|
| THE HAGUE | 13-01-1986 | BRINKMANN C. |

European Patent
Office

**EUROPEAN SEARCH REPORT**

**0179583**

Application number

EP 85 30 7025

| | DOCUMENTS CONSIDERED TO BE RELEVANT | | Page 2 | |
|---|---|---|---|---|
| Category | Citation of document with indication, where appropriate, of relevant passages | Relevant to claim | CLASSIFICATION OF THE APPLICATION (Int Cl 4) | |
| A | DIE PHARMAZIE, vol. 37, no. 5, May 1982, pages 359-362, Berlin, DE; L.M. MORTADA: "Dissolution rates of oxyphenbutazone in surfactant solutions" * Page 359, abstract * | 1-9 | | |
| D,A | EP-A-0 012 523 (AMERICAN HOME PRODUCTS) * & US - A - 4 344 934 | 1-9 | | |
| | | | TECHNICAL FIELDS SEARCHED (Int Cl 4) | |

The present search report has been drawn up for all claims

| Place of search THE HAGUE | Date of completion of the search 13-01-1986 | Examiner BRINKMANN C. |
|---|---|---|